# EUROPEAN PATENT APPLICATION

(11) **EP 0 755 932 A1**
(43) Date of publication of application: **29.01.1997**
(21) Application number: 96305444.0
(22) Date of filing: 24.07.1996
(51) Int. Cl.: C07D 451/02, A61K 31/395

(54) **(Thiophen-2-yl)piperidin or tetrahydropyridin-azabicyclocarboxamides**

(30) Priority: 26.07.1995 US 1523
(71) Applicant: AMERICAN HOME PRODUCTS CORPORATION, Madison, New Jersey 07940-0874 (US)
(72) Inventor: Baudy, Reinhardt Bernhard, Yardley, Pennsylvania, 19067 (US); Nelson, James Albert, Washington Crossing, Pennsylvania, 18977 (US); Kanzelberger, Mira Ana, Monmouth Junction, New Jersey, 08852 (US)
(74) Representative: Wileman, David Francis, Dr.

(57) **Abstract**

This invention provides compounds having the structure 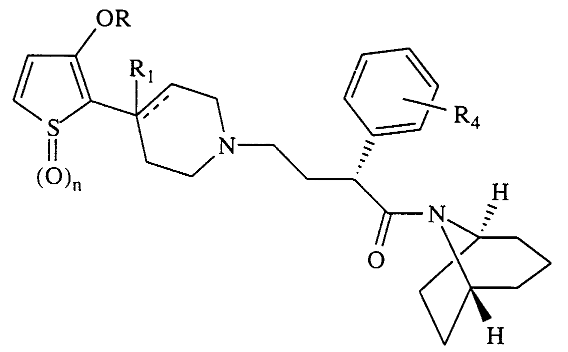
wherein
R is hydrogen, alkyl, alkenyl, alkynyl, -COR², phenyl, or phenylalkyl; the dotted line represents an optional double bond;
R¹ is hydrogen, -OH, OR³, or is absent if the optional double bond is present;
R² and R³ are each, independently, alkyl, alkenyl, alkynyl, phenyl, orphenylalkyl;
R⁴ is hydrogen, -OR⁵, alkyl, alkenyl, alkynyl, -COR⁵, -CO₂R⁵, -CONR⁵R⁶, perhaloalkyl, halogen, phenyl, or phenylalkyl;
R⁵ and R⁶ are each, independently, hydrogen, alkyl, alkenyl, alkynyl, phenyl, or phenylalkyl; and
n = 0 - 2
or a pharmaceutically acceptable salt thereof that are useful as antipsychotic, antidepressant and anxiolytic agents useful in the treatment and relief of the symptoms of these disease states.

## Description

The present invention relates to the provision of compounds useful in the treatment of central nervous system disorders, in particular to the provision of (thiophen-2-yl)-piperidin or tetrahydropyridin azabicyclocarboxamides having selectivity for the serotonergic 5-HT_{1A} receptor.

This invention provides compounds having a structure of Formula IV 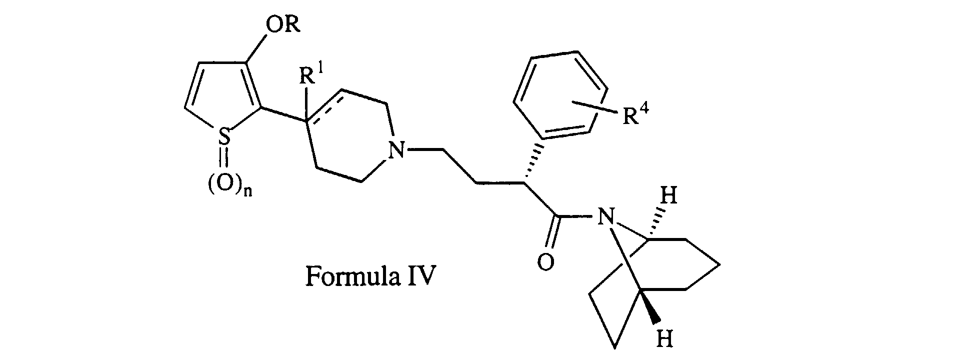 wherein
- R: is hydrogen, alkyl of 1-6 carbon atoms, alkenyl of 2-7 carbon atoms, alkynyl of 2-7 carbon atoms, -COR², phenyl, or phenylalkyl of 7-10 carbon atoms;
the dotted line represents an optional double bond;
- R¹: is hydrogen, -OH, OR³, or is absent if the optional double bond is present;
- R² and R³: are each, independently, alkyl of 1-6 carbon atoms, alkenyl of 2-7 carbon atoms, alkynyl of 2-7 carbon atoms, phenyl, or phenylalkyl of 7-10 carbon atoms;
- R⁴: is hydrogen, -OR⁵, alkyl of 1-6 carbon atoms, alkenyl of 2-7 carbon atoms, alkynyl of 2-7 carbon atoms, -COR⁵, -CO₂R⁵, -CONR⁵R⁶, perhaloalkyl of 1-6 carbon atoms, halogen, phenyl, or phenylalkyl of 7-10 carbon atoms;
- R⁵ and R⁶: are each, independently, hydrogen, alkyl of 1-6 carbon atoms, alkenyl of 2-7 carbon atoms, alkynyl of 2-7 carbon atoms, phenyl, or phenylalkyl of 7-10 carbon atoms; and
- n =: 0 - 2
or a the group thereof.

The pharmaceutically acceptable salts are those derived from such organic and inorganic acids as: acetic, lactic, citric, tartaric, succinic, maleic, malonic, gluconic, hydrochloric, hydrobromic, phosphoric, nitric, sulfuric, methanesulfonic, and similarly known acceptable acids.

The terms alkyl of 1-6 carbon atoms, alkenyl of 2-7 carbon atoms, and alkynyl of 2-7 carbon atoms, include both straight chain as well as branched carbon chains. In the generic structure described above, when n = 0, the sulfur containing ring is a thiophene ring, when n = 1, the sulfur containing ring is a thiophene S-oxide, and when n = 2, the sulfur containing ring is a thiophene S-dioxide. The term "halogen" refers to fluoro, chloro, bromo, or iodo.

The compounds within the scope of the invention by virtue of their configuration, exhibit stereoisomerism. Such centers can contain either the R or S configuration or can be racemic with respect to such center or centers. Accordingly, the compounds of the invention include the diastereomers, enantiomers, racemates and mixtures thereof.

Standard separation techniques may be used to isolate particular enantiomeric and diastereomeric forms. For example a racemic mixture may be converted to a mixture of optically active diastereoisomers by reaction with a single enantiomer of a 'resolving agent' (for example by diastereomeric salt formation or formation of a covalent bond). The resulting mixture of optically active diastereoisomers may be separated by standard techniques (e.g crystallisation or chromatography) and individual optically active diastereoisomers then treated to remove the 'resolving agent' thereby releasing the single enantiomer of the compound of the invention. Chiral chromatography (using a chiral support, eluent or ion pairing agent) may also be used to separate enantiomeric mixtures directly.

The alkyl group (on its own or as part of a group, e.g. phenylalkyl or perhaloalkyl) is for example methyl, ethyl, isopropyl, n-propyl, n-butyl, t-butyl, s-butyl, isobutyl, n-pentyl or n-hexyl. The alkenyl group is for example ethenyl, 1-propenyl or 2-propenyl. The alkynyl group is for example ethynyl, or propynyl. The phenylalkyl group is for example phenylmethyl, phenylethyl, 2-phenylpropyl, 3-phenylpropyl or phenylbutyl. The perhaloalkyl group is for example an alkyl group substituted by fluoro or chloro groups e.g. trifluoromethyl, pertluoroethyl, perfluoro-n-propyl, perfluoro-i-propyl, perfluoro-n-butyl, perfluoro-i-butyl, perfluoro-s-butyl, perfluoro-t-butyl, perfluoro-n-pentyl, trichloromethyl or trifluorodichloroethyl.

R¹ is preferably hydrogen or -OH. R is preferably methyl.

Prefered compounds of the invention are those in which n = 0; and those in which n = 0, and R is alkyl of 1-6 carbon atoms.

Particular compounds of the invention are (R)-1-(8-aza-bicyclo[3.2.1.]oct-8-yl)-4-[4-hydroxy-4-(3-methoxy-thiophen-2-yl)-piperidin- 1 -yl]-2-phenyl-butan- 1-one, (R)-1-(8-Aza-bicyclo[3.2.1.]oct-8-yl)-4-[4-(3-methoxy-thiophen-2-yl)-1,2,3,6-tetrahydropyridin-1-yl]-2-phenyl-butan-1-one and pharmaceutically acceptable salts thereof, in particular (R)-1-(8-Aza-bicyclo[3.2.1.]oct-8-yl)-4-[4-hydroxy-4-(3-methoxy-thiophen-2-yl)-piperidin-1-yl]-2-phenyl-butan-1-one hydrochloride salt and (R)-1-(8-Azabicyclo[3.2.1]oct-8-yl)-4-[4-(3-methoxy-thiophen-2-yl)-1,2,3,6-tetrahydro-pyridin-1-yl]-2-phenyl-butan-1-one hemi-hydrochloride.

The present invention further provides a method of treating anxiety, psychosis, or depression in a mammal in need thereof which comprises administering to said mammal, an effective amount of a compound of Formula IV.

The present invention further provides a pharmaceutical composition which comprises a compound of Formula IV or a pharmaceutically acceptable salt thereof and a pharmaceutical carrier.

The present invention also provides a method for the preparation of a compound of Formula IV or a pharmaceutically acceptable salt thereof comprising
a) subjecting a 4-substituted piperidine of Formula II 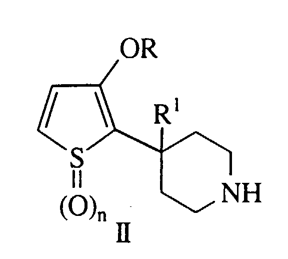 wherein R and n are as defined above and R¹ is OH or OR³ (R³ is as defined above) to reductive amination by reacting it with a substituted butyraldehyde of Formula IIa 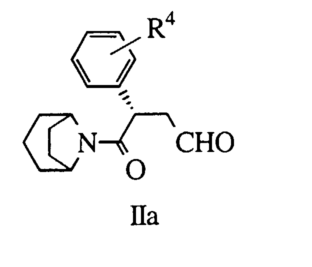 to yield a compound of Formula IV wherein R¹ is OH (this is preferably performed in the presence of sodium borocyanohydride),
or b) functionalising a compound of Formula IV wherein R¹ is OH to give a compound of Formula IV wherein R¹ is OR³ (e.g. by treating the hydroxy compound with a strong base such as butyl lithium or lithium diisopropylamide, followed by an organohalide containing R³),
or c) dehydrating a compound of Formula IV wherein R¹ is OH to provide the corresponding tetrahydropyridine derivative (this is preferably performed under mildly acidic conditions)
or d) hydrogenating a compound of Formula IV wherein the optional bond is present to give a compound of Formula IV wherein R¹ is H,
or e) reacting a compound of Formula IV wherein R¹ is OH with an alkylsulphonylhalide (e.g. methanesulphonyl chloride) followed by treatment with lithium aluminium hydride to give a compound of Formula IV wherein R¹ is H,
or f) after any of the aforementioned steps isolating the compound of Formula IV as a pharmaceutically acceptable salt.
One example of the methods of the invention is to subject an appropriately substituted 2-bromo-thiophene to a Grignard reaction in which the addition to a 4-piperidone carbamate affords the desired tertiary alcohol. Subsequent hydrolysis of the carbamate yields the desired 4-hydroxy-4-thiophen-2-yl-piperidine as shown in scheme 1. 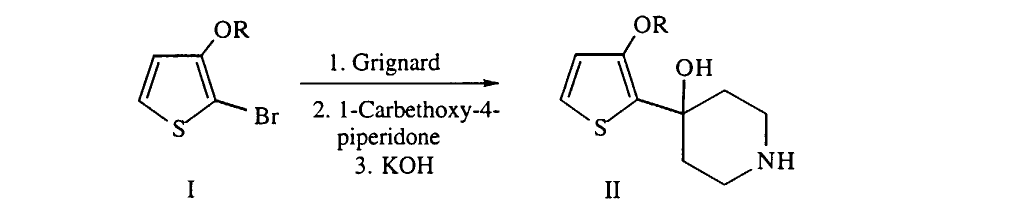 The 4-substituted piperidine is subjected to a reductive amination using the substituted butyraldehyde in the presence of sodium borocyanohydride to yield the final products as illustrated in scheme 2. 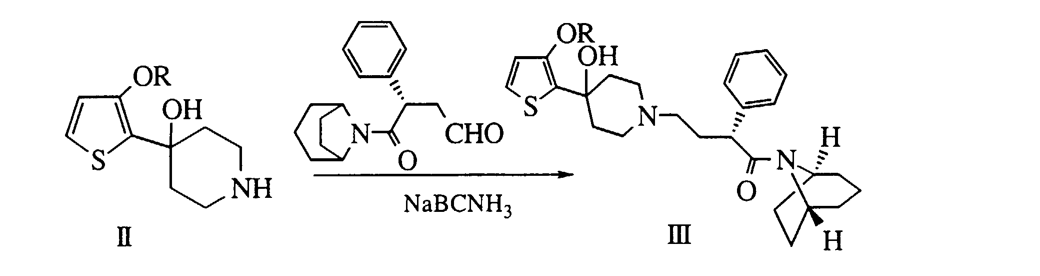 Further functionalization of the piperidine hydroxyl group can be accomplished using standard methodology, and dehydration of the hydroxyl group to provide tetrahydropyridine derivatives can be accomplished under mildly acidic conditions.

Representative compounds of this invention were evaluated and determined to have high affinity for the serotonin 5-HT_{1A} receptor by evaluating the compound's ability to displace [³H] 8-OHDPAT (dipropylaminotetralin) from the 5-HT_{1A} serotonin receptor following the procedure of Hall et al., J. Neurochem. 44, 1685 (1985). This standard pharmacological test procedure was employed to analogize this property of the claimed compounds with that of buspirone, which is a standard for anxiolytic activity, and. like the compounds of this invention, displays potent affinity for the 5-HT_{1A} serotonin receptor subtype. The anxiolytic activity of buspirone is believed to be, at least partially, due to its 5-HT_{1A} receptor affinity (Vander Maclen et al., Eur. J. Pharmacol. 1986, 129 (1-2) 133-130). In this standard pharmacological test procedure, buspirone has an IC₅₀ of approximately 10 nM.

The results obtained for representative compounds of this invention in the standard pharmacological test procedure described above, are as follows:

| Compound | 5-HT_{1A} Binding (IC₅₀) |
|---|---|
| Example 1 | 942 nM |
| Example 2 | 0.9 nM |

The results obtained in the standard pharmacological test procedure demonstrate that the compounds this invention possess high affinities for the serotonin 5-HT_{1A} receptor, and consequently, they are useful in the treatment of multi-CNS disorders amenable to treatment with antipsychotic, antidepressant and anxiolytic agents. As such, the compounds of this invention may be administered a mammal in need of antipsychotic, antidepressant and/or anxiolytic medical treatment in an amount sufficient to alleviate the symptoms of the disease state, such as depression, paranoia, schizophrenia, anxiety, sleep disorders, eating disorders, cognitive disorders, panic, social phobia, obsessive compulsive disorders, sexual dysfunction, addiction, and related problems. When administered for the treatment of the above disease states, the compounds of this invention can be administered to a mammal orally, parenterally, intranasally, intrabronchially, transdermally, intravaginally, or rectally.

The compounds of this invention can be formulated neat or with a pharmaceutical carrier to a mammal in need thereof. The pharmaceutical carrier may be solid or liquid.

A solid carrier can include one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration. The liquid carrier for pressurized compositions can be halogenated hydrocarbon or other pharmaceutically acceptable propellant.

Liquid pharmaceutical compositions which are sterile solutions or suspensions can be utilized by, for example, intramuscular, intraperitoneal or subcutaneous injection. Sterile solutions can also be administered intravenously. The compound can also be administered orally either in liquid or solid composition form.

The compounds of this invention may be administered rectally in the form of a conventional suppository. For administration by intranasal or intrabronchial inhalation or insufflation, the compounds of this invention may be Formulated into an aqueous or partially aqueous solution, which can then be utilized in the form of an aerosol. The compounds of this invention may also be administered transdermally through the use of a transdermal patch containing the active compound and a carrier that is inert to the active compound, is non toxic to the skin, and allows delivery of the agent for systemic absorption into the blood stream via the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may also be suitable. A variety of occlusive devices may be used to release the active ingredient into the blood stream such as a semipermiable membrane covering a reservoir containing the active ingredient with or without a carrier, or a matrix containing the active ingredient. Other occlusive devices are known in the literature.

The dosage to be used in the treatment of a specific psychosis must be subjectively determined by the attending physician. The variables involved include the specific psychosis or state of anxiety or depression and the size, age and response pattern of the patient. Based on the results obtained in the standard pharmacological test procedures, projected oral daily dosages of active compound would be 1-100 mg/kg, preferably between 1-30 mg/kg, and more preferably between 1-10 mg/kg. Projected intravenous daily dosages would be 0.2-20 mg/kg, preferably between 0.2-6 mg/kg, and more preferably between 0.2-2 mg/kg. Treatment will generally be initiated with small dosages less than the optimum dose of the compound. Thereafter the dosage is increased until the optimum effect under the circumstances is reached; precise dosages for oral, parenteral, nasal, or intrabronchial administration will be determined by the administering physician based on experience with the individual subject treated.

Preferably, the pharmaceutical composition is in unit dosage form, e.g. as tablets or capsules. In such form, the composition is sub-divided in unit dose containing appropriate quantities of the active ingredient; the unit dosage forms can be packaged compositions, for example, packeted powders, vials, ampoules, prefilled syringes or sachets containing liquids. The unit dosage form can be, for example, a capsule or tablet itself, or it can be the appropriate number of any such compositions in package form.

The following examples illustrate the production of representative compounds of this invention.

### Preparation of (R)-4-(8-aza-bicyclo[3.2.1.]oct-8-yl)-4-oxo-3-phenylbutyraldehyde

The starting (R)-2-phenyl-4-pentenoic acid can be prepared according to the procedure of Cervinka et. al.: Collect. Czech. Chem. Commun. (1967), 32(6), 2295-300.

The reagent nortropane was prepared according to established methods, e.g. J. Org. Chem., (1984), 49, 2081-2.

Under dry nitrogen and protected from light l-hydroxybenzotriazole (0.46 g, 2.84 mmole) was added to a solution of the starting (R)-2-phenyl-4-pentenoic acid (0.5 g, 2.84 mmole) in methylene chloride (5 mL) at ambient temperature, followed by adding a solution of 1-(3-dimethylaminopropyl)-3-ethylcarbodiimide hydrochloride (0.54 g, 2.84 mmole) in methylene chloride (20 mL). The reaction mixture was stirred for 2 hours after which a solution of nortropane (0.32 g, 2.84 mmole) in methylene chloride (5 mL) was added dropwise and stirring continued at room temperature overnight. The solvent was removed in vacuo at ambient temperature and the residue was partitioned between ethyl acetate (20 mL) and 1N hydrochloric acid (20 mL). The separated organic layer was washed with water (4x 20 mL), brine (2x 20 mL), dried over magnesium sulfate and filtered. The filtrate was concentrated in vacuo to yield the desired crude (R)-1-(8-aza-bicyclo[3.2.1.]oct-8-yl)-2-phenyl-pent-4-en-1-one which may be purified by flash chromatography on 60 g silica gel using 35% ethyl acetate / hexane as eluant.
The material (0.5 g, 1.86 mmole) was dissolved in a mixture of tetrahydrofuran (13 mL) and water (4 mL) and stirred under nitrogen. Osmium tetroxide (0.37 mL, 0.03 equivalents as a 4% aqueous solution) was added followed by portionwise addition of sodium periodate (1.19 g, 5.56 mmole). After stirring at ambient temperature for 1 hour, water (10 mL), ethyl acetate (20 mL), and brine (10 mL) were added to the reaction mixture. The organic layer was separated, washed with brine (2x 20 mL), dried over magnesium sulfate, and filtered. The filtrate was evaporated in vacuo. Column flash chromatography of the residue on 52 g of silica gel with 50% ethyl acetate / hexane as eluant, followed by crystallization from ether / hexane yields 0.29 g of the title compound, m.p. 82-5 °C.

### Example 1: (R)- 1-(8-Aza-bicyclo[3.2.1.]oct-8-yl)-4-[4-hvdroxy-4-(3-methoxythiophen-2-yl)-piperidin-1-yl]-2-phenylbutan-1-one

Acetic acid (0.48 mL) was added to a solution of the starting 4-hydroxy-4-thiophen-2-yl-piperidine (0.853 g, 4 mmole) and (R)-4-(8-aza-bicyclo[3.2.1.]oct-8-yl)-4-oxo-3-phenyl-butyraldehyde (1.085 g, 4 mmole) in methanol (30 mL) at ambient temperature. Thereafter sodium cyanoborohydride (0.276 g, 4.4 mmole) was added at once and the reaction mixture stirred at room temperature for 3 hours. The mixture was poured into 10% aqueous sodium bicarbonate (60 mL) and extracted with methylene chloride (3x 50 mL). The organic extracts were combined, washed with water (50 mL), brine (50 mL), dried over magnesium sulfate and filtered. The filtrate was evaporated in vacuo and the residue dissolved in chloroform (50 mL). Ethanolic hydrochloric acid was added and the resulting solution evaporated. The residue was triturated in ether and the precipitate filtered and dried to yield 1.8 g of the hydrochloride salt of the title compound, mp 106-8°C.

| Elemental Analysis for: C₂₇H₃₆N₂O₃S · HCl. | | | |
|---|---|---|---|
| Calcd: | C, 64.20; | H, 7.38; | N, 5.55. |
| Found: : | C, 64.08; | H, 7.57; | N, 5.40. |

### Example 2: (R)-1-(8-Aza-bicyclo[3.2.1.]oct-8-yl)4-[4-(3-methoxythiophen-2-yl)-1,2,3,6-tetrahydropyridin-1-yl]-2-phenylbutan-1-one

The starting (R)-1-(8-Aza-bicyclo[3.2.1.]oct-8-yl)-4-[4-hydroxy-4-(3-methoxy-thiophen-2-yl)-piperidin-1-yl]-2-phenyl-butan-1-one hydrochloride (example 1, 1.7 g, 3.6 mmole) was dissolved in acetic acid (80 mL) and heated to 140°C bath temperature for 2 ¹/₂ hours. Thereafter the reaction mixture was stirred at room temperature overnight, evaporated to dryness in vacuo and the residue partitioned between 5% aqueous sodium bicarbonate (50 mL) and chloroform (2x 100 mL). The combined organic layer was separated, dried over magnesium sulfate, filtered, and evaporated to dryness in vacuo. Column chromatography on 100 g of silica gel with 3% methanol / chloroform as eluant, followed by crystallization from ethanol/ether with addition of ethanolic hydrochloric acid yields 0.7 g of the hemihydrochloride of the title compound, m.p. 100-3 °C.

| Elemental Analysis for: C₂₇ H₃₄ N₂ O₂ S · 1.5 HCl | | | |
|---|---|---|---|
| Calcd: | C, 64.17; | H, 7.08; | N, 5.54. |
| Found: : | C, 64.02; | H, 6.94; | N, 5.32. |

## Claims

1. A compound of the structure: 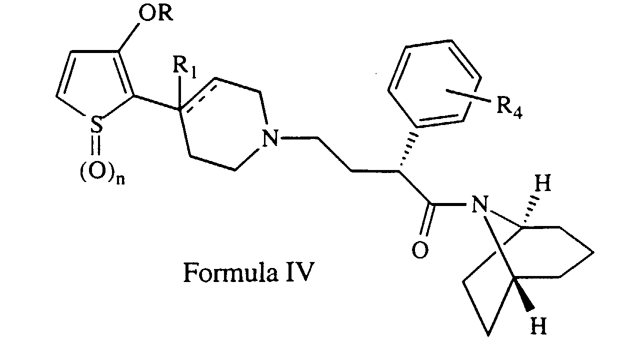 wherein
R is hydrogen, alkyl of 1-6 carbon atoms, alkenyl of 2-7 carbon atoms, alkynyl of 2-7 carbon atoms, -COR², phenyl, or phenylalkyl of 7-10 carbon atoms;
the dotted line represents an optional double bond;
R¹ is hydrogen, -OH, OR³, or is absent if the optional double bond is present;
R² and R³ are each, independently, alkyl of 1-6 carbon atoms, alkenyl of 2-7 carbon atoms, alkynyl of 2-7 carbon atoms, phenyl, or phenylalkyl of 7-10 carbon atoms;
R⁴ is hydrogen, -OR⁵, alkyl of 1-6 carbon atoms, alkenyl of 2-7 carbon atoms, alkynyl of 2-7 carbon atoms, -COR⁵, -CO₂R⁵, -CONR⁵R⁶, perhaloalkyl of 1-6 carbon atoms, halogen, phenyl, or phenylalkyl of 7-10 carbon atoms;
R⁵ and R⁶ are each, independently, hydrogen, alkyl of 1-6 carbon atoms, alkenyl of 2-7 carbon atoms, alkynyl of 2-7 carbon atoms, phenyl, or phenylalkyl of 7-10 carbon atoms; and
n = 0 - 2
or a pharmaceutically acceptable salt thereof.

2. The compound of Claim 1, wherein n = 0 or a pharmaceutically acceptable salt thereof.

3. The compound of Claim 2, wherein R is alkyl of 1-6 carbon atoms or a pharmaceutically acceptable salt thereof.

4. The compound of Claim 1 which is (R)-1-(8-aza-bicyclo[3.2.1.]oct-8-yl)-4-[4-hydroxy4-(3-methoxy-thiophen-2-yl)-piperidin-1-yl]-2-phenyl-butan-1-one or a pharmaceutically acceptable salt thereof.

5. The compound of Claim 1 which is (R)-1-(8-Aza-bicyclo[3.2.1.]oct-8-yl)-4-[4-hydroxy4-(3-methoxy-thiophen-2-yl)-piperidin-1-yl]-2-phenyl-butan-1-one hydrochloride salt.

6. The compound of Claim 1 which is (R)-1-(8-Aza-bicyclo[3.2.1.]oct-8-yl)-4-[4-(3-methoxy-thiophen-2-yl)-1,2,3,6-tetrahydro-pyridin-1-yl]-2-phenyl-butan-1-one or a pharmaceutically acceptable salt thereof.

7. The compound of Claim 1 which is (R)-1-(8-Aza-bicyclo[3.2.1.]oct-8-yl)-4-[4-(3-methoxy-thiophen-2-yl)-1,2,3,6-tetrahydro-pyridin-1-yl]-2-phenyl-butan-1-one hemi-hydrochloride.

8. A method of treating anxiety, psychosis, or depression in a mammal in need thereof which comprises administering to said mammal, an effective amount of a compound as claimed in Claim 1.

9. A pharmaceutical composition which comprises a compound as claimed in Claim 1 and a pharmaceutically acceptable carrier.

10. A method for the preparation of a compound of Formula IV or a pharmaceutically acceptable salt thereof comprising:
a) subjecting a 4-substituted piperidine of Formula II 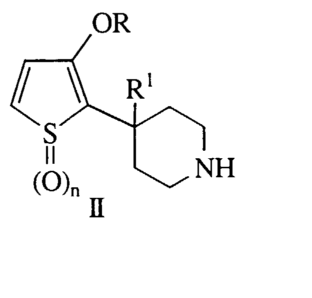 wherein R and n are as defined in Claim 1 and R¹ is OH to reductive amination by reacting it with a substituted butyraldehyde of Formula IIa 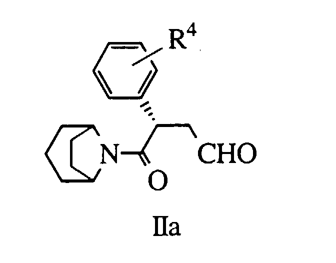 to yield a compound of Formula IV wherein R¹ is OH or OR³,
or b) functionalising a compound of Formula IV wherein R¹ is OH to give a compound of Formula IV wherein R¹ is OR³,
or c) dehydrating a compound of Formula IV wherein R¹ is OH to provide the corresponding tetrahydropyridine derivative,
or d) hydrogenating a compound of Formula IV wherein the optional bond is present to give a compound of Formula IV wherein R¹ is H,
or e) reacting a compound of Formula IV wherein R¹ is OH with an alkylsulphonylhalide followed by treatment with lithium aluminium hydride to give a compound of Formula IV wherein R¹ is H,
or f) after any of the aforementioned steps isolating the compound of Formula IV as a pharmaceutically acceptable salt.
